**Europäisches Patentamt**

**European Patent Office**

**Office européen des brevets**

(19)

(11) Publication number : **0 330 688 B1**

## EUROPEAN PATENT SPECIFICATION

(12)

(45) Date of publication of patent specification :
13.09.95 Bulletin 95/37

(51) Int. Cl.⁶ : **G01N 33/569**

(21) Application number : **88907165.0**

(22) Date of filing : **28.07.88**

(86) International application number :
**PCT/AU88/00274**

(87) International publication number :
**WO 89/01162 09.02.89 Gazette 89/04**

(54) DETECTION METHODS.

(30) Priority : **28.07.87 AU 3384/87**

(43) Date of publication of application :
**06.09.89 Bulletin 89/36**

(45) Publication of the grant of the patent :
**13.09.95 Bulletin 95/37**

(84) Designated Contracting States :
**AT BE CH DE FR GB IT LI LU NL SE**

(56) References cited :
**EP-A- 0 201 211
WO-A-84/02193
AU-A- 5 318 786
AU-A- 6 659 686
FR-A- 2 514 367
US-A- 4 434 236
US-A- 4 752 562
BULLETIN OEPP/EPPO BULLETIN, vol. 17,
1987, Wageningen (NL); J.W.L. VAN VUURDE,
pp. 139-148/
JOURNAL OF MEDICAL MICROBIOLOGY, vol.
8, 1975, US; B. MOHIT et al., pp. 173-176/**

(73) Proprietor : **BIOTECHNOLOGY AUSTRALIA
PTY. LTD.
28 Barcoo Street
East Roseville, NSW 2069 (AU)**

(72) Inventor : **ATRACHE, Vincent, Habib
4 Benaroon Avenue
St. Ives, NSW 2075 (AU)**
Inventor : **ASH, Megan
20/3-7 Edgeworth David Avenue
Hornsby, NSW 2077 (AU)**
Inventor : **VAN HUYNH, Ca
67 Blaxcell Street
Granville, NSW 2142 (AU)**

(74) Representative : **Bannerman, David Gardner et
al
Withers & Rogers
4 Dyer's Buildings
Holborn
London, EC1N 2JT (GB)**

EP 0 330 688 B1

## Description

Technical Field

The current invention relates to methods for detecting low levels of a particular microorganism, or microorganisms from a mixed culture or sample using antibodies and solid immunosorbent supports without the need for a preliminary or further growth step in selective media.

Background Art

Solid-phase immunoassays, based either on enzymes or radioactive isotopes as labels have found wide application in diagnostic microbiology due to their high specificity and sensitivity.

The specificity of an immunoassay is determined by the antibody or antigen which has been immobilized on the solid support. A major advantage of a solid phase assay is that on completion of the immune reaction, unwanted material is easily and rapidly separated from the antigen-antibody immune complex by a simple washing step. A wide variety of solid supports have found application for antibody or antigen immobilization and include polystyrene, polyvinyl chloride, nylon, titanous hydroxide, agarose beads and nitrocellulose.

The successful application of immunoassays to the detection of microorganisms in a sample is possible only if the particular organism of interest is present in sufficient numbers. This critical concentration is determined by the sensitivity of the immunoassay which can vary greatly depending on the affinity and avidity of a particular antibody for its antigen. It is for this reason that many immunoassays require culturing of the sample prior to performing the test.

This usually involves a pre-enrichment step to resuscitate injured microorganisms followed by a selective enrichment to increase the numbers of the microorganism of interest. Selecting culture conditions which favour the growth of a particular microorganism over its competitors has traditionally involved the use of either antibiotics, specific nutritional requirements or manipulation of the physical characteristics of the growth medium, e.g. temperature. These methods can take one to two days or several weeks depending on the organism and the extent of contamination with other microflora.

The use of immunosorbents for selecting microorganims from a mixed population is known. The sorbent immobilizes a predetermined species of microorganism against which the antibodies are directed. Cells captured in this way can be incubated in a growth medium and then counted by such traditional techniques as plating and colony counting.

US Patent 4 592 994 describes a method for the determination or identification of microorganisms or unicellular organisms in a sample. The method involves exposing the sample to an absorbent having "a specific binding power" which may be provided by an antibody raised against the microorganism to be detected. Unbound sample is separated and the adsorbent with bound organisms is exposed to a nutrient medium to initiate metabolism. This nutrient medium undergoes physical or chemical changes as a result of this metabolism and these changes are observed in conjunction with calibration curves to determine presence and amount of the relevant microorganism. The assay involves indirect detection of original numbers of organisms through detection of metabolites in the medium. This may generate problems with regard to the specificity of the assay since different microorganisms may share metabolites.

US Patent 4 563 418 describes a method for the detection of a particular motile organism in a sample, for example, flagellate bacteria such as Salmonella species.

The method involves enriching the sample in an enrichment medium selective for the particular motile organism and filling a motility vessel with a non-selective medium containing a chemotactic attractant which serves to temporarily immobilize the organism of interest and its competitors in the medium for some time after inoculation. Antibodies specific to the flagella of the particular motile microorganism are added through another opening in the motility vessel. The vessel is incubated under sufficient temperature and time conditions to permit the motile organisms to metabolize the chemotactic attractant, thereby reducing its concentration sufficiently to allow movement of the organisms present with the result that the organisms move through the medium and the particular motile organisms being assayed are immobilized by the antibodies. The quantity of antibody used is sufficient to produce a permanent immobilization band.

In Mohit et al ["A Simple Single-step Immunoimmobilization Method for the Detection of Salmonella in the Presence of Large Numbers of Other Bacteria" J. Med. Microbiol. 8 173 (1975)], a method of detecting Salmonella in a mixed population is described. This method employs a selective semi-solid medium, which promotes the migration of Salmonella, followed by immobilization using polyvant H antisera.

La Roche et al in "Field Evaluation of the Membrane Filter-Disc Immobilization Technique in the Detection of Salmonella in Egg Products" describes a method for detecting Salmonella which involves using a membrane filter to concentrate Salmonella from a primary enrichment broth before selective migration in order to increase recovery.

Stannard reported in the annual report for Leatherhead RA for 1986, investigations into separation of Salmonella from other organisms, with a view to reducing the time required for detection of Salmonella in samples.

This method relied on antibody-coated magnetic particles. However, it was found that the apparent enrichment of Salmonella over other closely related organisms was in fact due to differential affinity of these organisms to the glassware used in the experiment. Attempts to use this differential affinity for glass to enrich Salmonella were unsuccessful due to the lack of specificity of the effect.

J.W.L. Van Vuurde, in Bulletin OEPP/EPPO Bulletin, 17, 139-148 (1987) discloses absorbing bacteria onto, eg. petri dishes, using selective antibodies, washing away unbound bacteria and then desorbing the selected bacteria and plating them on a suitable medium.

Thus it can be seen that the prior art methods for detecting microorganisms from mixed populations provide means for detecting low numbers where enrichment in selective medium is used. Immunoimmobilization has been used but has not been shown to be effective for detecting low numbers in the absence of a such a selection on selective medium.

The current invention provides methods for detecting low numbers of a particular microrganism or microorganisms in a mixed population which overcome the need for pre-selection in selective media by using an immunoimmobilization technique followed by non-selective growth and immunoassay.

The methods can be especially usefully applied to detecting Salmonella and Listeria spp meeting the need for rapid, sensitive methods for their detection in mixed populations.

Description of the Invention

The invention provides methods for the rapid detection of a particular microorganism, or microorganisms, in the presence of competing micro-flora using specific antibodies adsorbed onto a solid support. The immobilized antibody is to an antigen of the microorganism which allows selective capture and immobilization of the desired microorganism without compromising its ability to replicate. Appropriate antibodies can be raised against surface antigens provided by such surface structures as flagella or lipopolysaccharides. Selective concentration of the desired microorganism or microorganisms onto the solid support allows rapid separation from competing microflora in the sample and is achieved by simply washing the solid support. The immobilized cells can then be transferred to a nutrient broth to allow replication during which time the multiplying microorganisms will continue to be captured and immobilized on the solid support until the antibody sites are saturated.

The time taken for the concentration of the microorganisms to reach a detectable level will depend on the generation time of the particular microorganism or microorganisms and on the sensitivity limits of the immunoassay.

Once a detectable level is reached the solid support is simply separated from the culture broth, then washed and assayed directly.

This technique thus eliminates the need for elaborate and expensive enrichment media in providing rapid sensitive detection of low levels of a particular microorganism or microorganisms from mixed populations.

In a first embodiment, the invention provides a method for detecting low levels of a particular microorganism or microorganisms in the presence of competing microflora in a sample which method comprises: exposing the sample to a solid support to which are adsorbed antibodies specific for the microorganism or microorganisms being detected, said antibodies being capable of selective capture and immobilisation of the microorganism or microorganisms without compromising the ability of the microorganism or microorganisms to replicate; washing the support to remove unbound materials; adding sterile nutrient broth to the support; incubating the support in the nutrient broth at a temperature and for a time relative to the generation time of the microorganism or microorganisms sufficient to allow the microorganism or microorganisms bound to the support to reach a detectable level; washing the support and then performing an immunoassay on the support using an immunoreagent specific for the microorganism or microorganisms being detected.

Preferably the antibody is raised against a surface antigen of the microorganism. More preferably the surface antigen is a flagellar protein or lipopolysaccharide.

In a preferred form, the antibodies are immobilized onto a solid support comprising a bead, tube or well.

Preferably, the support material is polystyrene, polyvinyl chloride, nylon, titanous hydroxide, agarose beads or nitrocellulose.

Preferred microorganisms according to the invention include Salmonella species and Listeria species.

The exposure of the sample to the antibody-coated support is generally for 1 hour or less. The wash steps are preferably performed using sterile saline buffer, more preferably Tris-saline buffer. Virtually any nutrient broth may be used in the method, however, preferred broths are tryptone soya broth and M broth. The incubation in nutrient medium may be overnight and is usually at 37°C. However, for detection of <u>Salmonella</u> <u>typhimurium</u>, incubation times of approximately 6 hours have been shown to be effective. Where higher numbers of the particular microorganism are present in the original sample incubation times of between 1 and 6 hours can be used. Preferably, the immunoassay is an ELISA.

In a more preferred form the method comprises: exposing a <u>Salmonella</u> test sample for between about 5 and about 30 minutes to a polystyrene support to which are adsorbed anti-<u>Salmonella</u> flagella antibodies, said antibodies being capable of selective capture and immobilisation of <u>Salmonella</u> without compromisinq the ability of the <u>Salmonella</u> to replicate; washing the support with a first wash solution to remove unbound material; adding sterile nutrient broth to the support; incubating the support at 37°C for between about 1 and about 6 hours; washing the support with a second wash solution; adding an enzyme-labelled antibody specific to <u>Salmonella</u>; incubating for between about 5 and about 30 minutes; discarding any excess enzyme-labelled antibody; washing the support; adding a chromogenic substrate specific for the enzyme of the enzyme-labelled antibody; and measuring conversion of the chromogenic substrate to a coloured compound.

Preferably the support is a tube, bead or microtitre well.

Preferably the enzyme-labelled antibody is an (anti-<u>Salmonella</u>) antibody - peroxidase conjugate. However, it is recognised that it is possible to use an unlabelled anti-<u>Salmonella</u> antibody in conjunction with a labelled antibody raised against the anti-<u>Salmonella</u> antibody in an indirect version of the assay for bound <u>Salmonella</u>.

Preferably the wash is performed using a Tris-saline solution as wash solution.

Preferably the substrate is an ABTS/$H_2O_2$ (2,2'-azinobis(3-ethylbenzthiazoline sulfonic acid)/hydrogen peroxide) solution.

Preferably the nutrient broth is tryptone soya broth. Tryptone Soya Broth comprises:

|  | grams per litre |
| --- | --- |
| Casein Peptone, Pancreatic Digest (RM 271) | 17.0 |
| Soy Peptone (RM 322) | 3.0 |
| di-Potassium Phosphate | 2.5 |
| Sodium Chloride | 5.0 |
| Glucose | 2.5 |

pH 7.3 ± 0.2

The invention also provides a test kit for the detection of low levels of a particular microorganism or microorganisms in a mixed population which kit comprises: a solid support to which are adsorbed antibodies specific for the microorganism or microrganisms being detected; a wash solution; an enzyme-labelled antibody specific for the microorganism or microorganisms being detected; and a solution of a chromogenic substrate for the enzyme of the enzyme-labelled antibody.

In place of the enzyme-labelled antibody the kit may comprise an anti-microorganism antibody together with an enzyme-labelled antibody raised against the anti-microorganism antibody.

Best Method of Performing the Invention

The invention is further described with reference to the following examples which are in no way limiting on the scope of the invention.

Example 1

Selective isolation of Salmonella using antibody coated wells

<u>Salmonella</u> <u>typhimurium</u> (inhouse strain BTA 438) and <u>Citrobacter</u> <u>diversus</u> (BTA 1323) were inoculated into individual M broth solutions and were incubated overnight at 37°C.

M-Broth comprises the following:

|  | grams per litre |
|---|---|
| Casein Peptone Pancreatic Digest | 12.5 |
| Sodium Chloride | 5.0 |
| Sodium Citrate | 5.0 |
| Yeast Extract | 5.0 |
| D-Mannose | 2.0 |
| Ferrous Sulphate | 0.04 |
| Dipotassium Phosphate | 5.0 |
| Magnesium Sulphate | 0.8 |
| Manganese Chloride | 0.14 |
| Tween 80 (Trade Mark) | 0.75 |

## pH 7.0 ± 0.2

Each of the cultures grew to $10^9$ organisms/ml. These were then diluted as follows:

$10^8$ cells/ml C. diversus with $10^4$ cells/ml S. typhimurium

$10^8$ cells/ml C. diversus with $10^3$ cells/ml S. typhimurium

$10^8$ cells/ml C. diversus with $10^2$ cells/ml S. typhimurium

$10^8$ cells/ml C. diversus with $10^1$ cells/ml S. typhimurium

$10^8$ cells/ml C. diversus with $10^0$ cells/ml S. typhimurium

$10^8$ cells/ml C. diversus only (control)

Polystyrene wells coated with highly purified antibodies to Salmonella flagella were then added to the M broths containing the mixed cultures. Wells coated with non-immune sheep immunoglobulin were placed into a duplicate set of mixed cultures as a control. The broths containing the wells were shaken for 1 hour at 37°C.

The wells were then carefully removed and washed 3 times in Tris-saline, then placed in fresh M broth solutions. These M broths were incubated overnight at 37°C to allow the immobilised organisms to multiply. The wells were then removed from the M broths and an ELISA was performed on them.

### EIA Method

The wells were washed three times with Tris-saline-Tween (TST), then anti-Salmonella IgG - Horseradish Peroxidase labelled conjugate was added. After 30 minutes incubation at 37°C, the wells were washed three times with TST, then substrate (2,2-azinobis(3-ethylbenzthiazoline sulfonic acid)) in citrate-phosphate buffer (0.1M, pH4) containing hydrogen peroxide (0.005%) was added and colour allowed to develop.

| ORGANISMS/ML | | | OPTICAL DENSITY[1] | |
|---|---|---|---|---|
| C. diversus | | S. typhimurium | Salmonella Ab Coated Wells | Control Wells[2] |
| $10^8$ | + | $10^4$ | 2.0 | 0.026 |
| $10^8$ | + | $10^3$ | 1.378 | 0.035 |
| $10^8$ | + | $10^2$ | 0.306 | 0.063 |
| $10^8$ | + | $10^1$ | 0.314 | 0.042 |
| $10^8$ | + | $10^0$ | 0.066 | 0.050 |
| $10^8$ | | | 0.078 | 0.037 |

1. Optical densities read using dual wavelength ELISA reader at 414nm and 490nm.

2. Non-immune sheep immunoglobulin coated wells.

Example 2

The method was compared with the Standard Culture Enrichment protocol [AOAC Official Methods of Analysis 963-971 (1984)] and showed increased speed of performance, and enhanced selectivity and sensitivity as described below.

The protocol was as described in Example 1 except that 1ml of the M broth containing the mixed cultures was placed into 10ml of tetrathionate broth and incubated at 37°C for 6 hours.

Following this, an ELISA was performed on the broths. To the remaining 9ml of broth, wells coated with highly purified antibodies to Salmonella flagella were added and the procedure of Example 1 followed except that the incubation of the M broth was for 6 hours only and then an ELISA was performed.

| ORGANISMS/ML | | OPTICAL DENSITY[1] | |
|---|---|---|---|
| C.DIVERSUS | S.TYPHIMURIUM | SALMONELLA Ab COATED WELLS | TETRATHIONATE BROTHS |
| $10^8$ + | $10^4$ | 2.0 | 0.113 |
| $10^8$ + | $10^3$ | 2.0 | 0.089 |
| $10^8$ + | $10^2$ | 0.244 | 0.076 |
| $10^8$ + | $10^1$ | 0.200 | 0.061 |
| $10^8$ | control | 0.030 | 0.065 |

[1] Optical densities read using dual wavelength ELISA reader at 414nm and 490nm.

After 6 hours the ELISA detected $10^3$ Salmonella in the presence of $10^8$ Citrobacter from the coated wells whilst after 6 hours selective enrichment in tetrathionate no Salmonella were detected by the ELISA.

Example 3

Salmonella Rapid Detection Kit

This kit enables the user to rapidly test for the presence of Salmonella in food, environmental or clinical samples. Highly specific antibodies to Salmonella flagella coated onto the surface of polystyrene tubes are used to capture Salmonella organisms present in a test sample. This is achieved by incubating the test sample in the antibody coated tube for a short time e.g: 5-30 minutes. The tube is then washed thoroughly to remove unbound material. Sterile nutrient broth e.g: Tryptone soya broth is then added and the tube incubated at 37°C for 1-6h. During this time the immobilised Salmonella replicate and continue to be captured by available antibodies on the tube surface. This allows for a sufficient concentration of organisms to be reached for subsequent detection by immunoassay.

The presence of the captured organisms can now be detected by discarding the culture broth and then adding an enzyme-labelled antibody specific to Salmonella. After a short incubation (5-30 min) excess enzyme-antibody reagent is discarded and the tube washed.

The enzyme-antibody conjugate which has specifically bound to the immobilised organisms is detected by addition of a substrate for the enzyme.

Materials Provided

- anti-Salmonella antibody coated polystyrene tubes
- anti-Salmonella-antibody-Peroxidase conjugate
- wash solution Tris Saline Tween

- Substrate solution - ABTS/$H_2O_2$

By use of Listeria reagents in the place of the Salmonella reagents this kit can be adapted to the detection of Listeria.

The methods are more rapid than traditional culture enrichment protocols, with a positive result being obtainable as rapidly as 6 hours, compared to a minimum of 48 hours by standard culture methods.

Industrial Application

The current invention provides an alternative to use of specialized selection media for the detection of low numbers of a particular microorganism or microorganisms in a mixed population.

## Claims

1. A method for the detection of low levels of a particular microorganism or microorganisms in the presence of competing microflora in a sample which method comprises: exposing the sample to a solid support to which are adsorbed antibodies specific for the microorganism or microorganisms being detected, said antibodies being capable of selective capture and immobilisation of the microorganism or microorganisms without compromising the ability of the microorganism or microorganisms to replicate; washing the support to remove unbound materials; adding sterile nutrient broth to the support; incubating the support in the nutrient broth at a temperature and for a time, relative to the generation time of the microorganism or microorganisms, sufficient to allow the microorganism or microorganisms bound to the support to reach a detectable level; washing the support; and then performing an immunoassay on the support using an immuno-reagent specific for the microorganism or microorganisms being detected.

2. The method according to claim 1, wherein the antibodies are antibodies raised against a surface antigen of the microorganism or microorganisms.

3. The method according to claim 2, wherein the surface antigen is a flagellar protein or lipopolysaccharide.

4. The method according to any one of claims 1 to 3, wherein the solid support is a bead, tube or well.

5. The method according to claim 4, wherein the support is polystyrene, polyvinyl chloride, nylon, titanous hydroxide, agarose beads or nitrocellulose.

6. The method according to any one of claims 1 to 5, wherein the microorganism is a Salmonella species or a Listeria species.

7. The method according to any one of claims 1 to 6, wherein the sample is exposed to the support for one hour or less.

8. The method according to any one of claims 1 to 7, wherein the support is washed with a sterile saline buffer.

9. The method according to claim 8, wherein the sterile saline buffer is Tris-Saline buffer.

10. The method according to any one of claims 1 to 9, wherein the nutrient broth is tryptone soya broth or M broth.

11. The method according to any one of claims 1 to 10, wherein the incubation in nutrient broth is overnight at 37°C.

12. The method according to any one of claims 1 to 11, wherein the incubation in nutrient broth is at 37°C for 6 hours.

13. The method according to any one of claims 1 to 12, wherein the immunoassay is an ELISA.

14. The method according to claim 13, wherein the immunoreagent is an enzyme-labelled anti-microorganism

EP 0 330 688 B1

antibody.

15. The method according to claim 13, wherein the immunoreagent comprises an anti-microorganism antibody and an enzyme-labelled antibody raised against the anti-microorganism antibody.

16. The method according to any one of claims 1 to 14 which method comprises: exposing a Salmonella test sample for between about 5 and about 30 minutes to a polystyrene support, to which are adsorbed anti-Salmonella flagella antibodies, said antibodies being capable of selective capture and immobilisation of Salmonella without compromising the ability of the Salmonella to replicate; washing the support to remove unbound material with a first wash solution; adding sterile nutrient broth to the support; incubating the support at 37°C for between about 1 hour and about 6 hours; washing the support with a second wash solution; adding an enzyme-labelled antibody specific to Salmonella; incubating for between about 5 and about 30 minutes; discarding any excess enzyme-labelled antibody; washing the support with the second wash solution; adding a chromogenic substrate specific for the enzyme of the enzyme-labelled antibody; and measuring conversion of the chromogenic substrate to a coloured compound.

17. The method according to claim 16, wherein the support is a tube, bead or microtitre well.

18. The method according to claim 16 or 17, wherein the enzyme-labelled antibody is an (anti-Salmonella) antibody-peroxidase conjugate.

19. The method according to any one of claims 16 to 18, wherein the first wash is performed using a Tris-Saline solution as wash solution and the second wash is performed using a Tris-Saline-Tween solution as wash solution.

20. The method according to any one of claims 16 to 19, wherein the substrate is an $ABTS/H_2O_2$ solution.

21. The method according to any one of claims 16 to 20, wherein the nutrient broth is tryptone soya broth.

22. A test kit for the detection of low levels of a particular microorganism or microorganisms in a mixed population which kit comprises:
a solid support to which are adsorbed antibodies specific for the microorganisms being detected, said antibodies being capable of selective capture and immobilisation of the microorganism or microorganisms without compromising the ability of the microorganism or microorganisms to replicate; an enzyme-labelled antibody specific for the microorganism or microorganisms being detected; and a solution of a chromogenic substrate for the enzyme of the enzyme-labelled antibody.

23. The test kit according to claim 22, wherein the support comprises anti-Salmonella antibody coated polystyrene tubes.

24. The test kit according to claim 22 or claim 23, wherein the enzyme-labelled antibody comprises anti-Salmonella-antibody-Peroxidase conjugate.

25. The test kit according to claims 22 to 24, wherein the substrate solution comprises $ABTS/H_2O_2$.

26. A test kit for the detection of low levels of a particular microorganism or microorganisms in a mixed population which kit comprises:
a solid support to which are adsorbed antibodies specific for the microorganism or microorganisms being detected, said antibodies being capable of selective capture and immobilisation of the microorganism or microorganisms without compromising the ability of the microorganism or microorganisms to replicate; an antibody specific for the microorganism or microorganisms; an enzyme-labelled antibody specific for the antibody specific for the microorganism or microorganisms; and a solution of a chromogenic substrate for the enzyme of the enzyme-labelled antibody.

**Patentansprüche**

1. Eine Methode für das Aufspüren von geringen Mengen eines bestimmten Mikroorganismus oder Mikro-

8

organismen in Gegenwart von konkurrierenden Mikroflora in einer Probe, welche Methode besteht aus: die Probe einer festen Unterlage auszusetzen, an welche Antikörper spezifisch für den aufzuspürenden Mikroorganismus oder Mikroorganismen adsorbiert sind; diese Antikörper besitzen die Fähigkeit der selektiven Einnahme und Immobilisierung des Mikroorganismus oder Mikroorganismen ohne deren Fähigkeit zu reproduzieren zu kompromittieren; Waschen der Unterlage um ungebundene Materialen zu entfernen; die Unterlage in eine sterile Nährlösung zu begeben, bei einer bestimmten Temperatur und für eine bestimmte Dauer, abhängig von der Generationszeit des Mikroorganismus oder Mikroorganismen, ausreichend für den an die Unterlage gebundenen Mikroorganismus oder Mikroorganismen messbar zu werden; Waschen der Unterlage; und anschließend Ausführen einer Immunitätsprüfung an der Unterlage unter Verwendung eines Reagens spezifisch für den zu prüfenden Mikroorganismus oder Mikroorganismen.

2. Die Methode wie Beschrieben in Anspruch 1, wobei die Antikörper von einem Oberflächen-antigen des Mikroorganismus oder Mikroorganismen angehoben sind.

3. Die Methode wie Beschrieben in Anspruch 2, wobei das Oberflächen-antigen ein Geißelprotein oder ein Lipopolysaccharid ist.

4. Die Methode wie Beschrieben in Ansprüchen 1 bis 3, wobei die feste Unterlage eine Perle, Schlauch oder Schacht ist.

5. Die Methode wie Beschrieben in Anspruch 4, wobei die Unterlage Styropor, Polyvinylchlorid, Nylon, Titanhydroxid, Perlen oder Stickstoffzellulose ist.

6. Die Methode wie Beschrieben in Ansprüchen 1 bis 5, wobei der Mikroorganismus Salmonella oder Listeria ist.

7. Die Methode wie Beschrieben in Ansprüchen 1 bis 6, wobei die Probe der Unterlage für eine Stunde oder kürzer ausgesetzt ist.

8. Die Methode wie Beschrieben in Ansprüchen 1 bis 7, wobei die Unterlage mit einer sterilen Salzpufferlösung gewaschen wird.

9. Die Methode wie Beschrieben in Anspruch 8, wobei die sterile Salzpufferlösung Tris-Saline Puffer ist.

10. Die Methode wie Beschrieben in Ansprüchen 1 bis 9, wobei die Nährlösung Trypton-Soyalösung oder M-lösung ist.

11. Die Methode wie Beschrieben in Ansprüchen 1 bis 10, wobei das Ausreifen in der Nährlösung über Nacht bei 37 Grad Celsius stattfindet.

12. Die Methode wie Beschrieben in Ansprüchen 1 bis 11, wobei das Ausreifen in der Nährlösung für 6 Stunden bei 37 Grad Celsius stattfindet.

13. Die Methode wie Beschrieben in Ansprüchen 1 bis 12, wobei der Immunitätsprüfer ein ELISA ist.

14. Die Methode wie Beschrieben in Anspruch 13, wobei das Immunitätsreagens ein enzym-bezeichneter anti-Mikroorganismus Antikörper ist.

15. Die Methode wie Beschrieben in Anspruch 13, wobei das Immunitätsreagens einen anti-Mikroorganismus Antikörper und einen enzym-bezeichneten Antikörper, der gegen den anti-Mikroorganismus Antikörper angehoben ist, enthält.

16. Die Methode wie Beschrieben in Ansprüchen 1 bis 14, welche beinhaltet: das Aussetzen für zwischen 5 und etwa 30 Minuten einer Salmonella-probe auf eine Styroporunterlage, auf welche anti-Salmonella flagella Antikörper adsorbiert sind; diese Antikörper besitzen die Fähigkeit der selektiven Einnahme und Immobilisierung der Salmonella ohne deren Fähigkeit zu reproduzieren zu kompromittieren; Waschen der Unterlage mit einer ersten Waschlösung; Zufügung einer sterilen Nährlösung zu der Unterlage; Ausreifen der Unterlage bei 37 Grad Celsius für zwischen 1 und etwa 6 Stunden; Waschen der Unterlage mit einer zweiten Nährlösung; Zufügen eines enzym-bezeichneten Antikörpers spezifisch für Salmonella; Ausreifen für circa zwischen 5 und 30 Minuten; Entfernung jeglichen Überschusses an enzym-bezeichneten Antikörpern; und Messung der Umwandlung der chromogenischen Substrate zu einer farbigen Mischung.

17. Die Methode wie Beschrieben in Anspruch 16, wobei die Unterlage ein Schlauch, Perle oder Mikrotritierschacht ist.

18. Die Methode wie Beschrieben in Anspruch 16 oder 17, wobei der enzym-bezeichnete Antikörper ein (anti-Salmonella) Antikörper-peroxidase Konjugat ist.

19. Die Methode wie Beschrieben in Ansprüchen 16 bis 18, wobei das erste Waschen unter Verwendung einer Tris - Saline - Lösung ausgeführt wird, und das zweite Waschen unter Verwendung einer Tris - Saline - Tween Lösung ausgeführt wird.

20. Die Methode wie Beschrieben in Ansprüchen 16 bis 19, wobei das Substrat eine ABTS H2 O2 Lösung ist.

21. Die Methode wie Beschrieben in Ansprüchen 16 bis 20, wobei die Nährlösung eine Trypton-soyalösung ist.

22. Eine Testausrüstung für das Aufspüren von geringen Mengen eines Mikroorganismus oder Mikroorganismen in einer gemischeten Kultur, welche besteht aus: einer festen Unterlage, an welche Antikörper spezifisch für den aufzuspürenden Mikroorganismus oder Mikroorganismen adsorbiert sind; diese Antikörper besitzen die Fähigkeit der selektiven Einnahme und Immobilisierung des Mikroorganismus oder Mikroorganismen ohne deren Fähigkeit zu reproduzieren zu kompromittieren; ein enzym-bezeichneter Antikörper spezifisch für den aufzuspürenden Mikroorganismus oder Mikroorganismen; und eine Lösung eines chromogenischen Substrats für das Enzym des enzym-bezeichneten Antikörpers.

23. Die Testausrüstung wie Beschrieben in Anspruch 22, wobei die Unterlage anti Salmonella Antikörper-beschichtete Schläuche einschliesst.

24. Die Testausrüstung wie Beschrieben in Anspruch 22 oder 23, wobei der enzym-bezeichnete Antikörper anti - Salmonella - Antikörper - Peroxidase Konjugat enthält.

25. Die Testausrüstung wie Beschrieben in Ansprüchen 22 bis 24, wobei die Substratlösung ABTS H2 O2 enthält.

26. Eine Testausrüstung für das Aufspüren von geringen Mengen eines Mikroorganismus oder Mikroorganismen in einer gemischeten Kultur, welche besteht aus: einer festen Unterlage, an welche Antikörper spezifisch für den aufzuspürenden Mikroorganismus oder Mikroorganismen adsorbiert sind; diese Antikörper besitzen die Fähigkeit der selektiven Einnahme und Immobilisierung des Mikroorganismus oder Mikroorganismen ohne deren Fähigkeit zu reproduzieren zu kompromittieren; ein enzym-bezeichneter Antikörper spezifisch für den Mikroorganismus oder Mikroorganismen; und eine Lösung eines chromogenischen Substrats für das Enzym des enzymbezeichneten Antikörpers.

## Revendications

1. Procédé de détection de bas taux d'un microorganisme particulier ou de microorganismes en présence d'une microflore dans un échantillon , un procédé comprenant: l'exposition de l'échantillon à un support solide auxquels sont adsorbés des anticorps spécifiques contre un microorganisme ou des microorganismes étant détectés, lesdits anticorps étant capables de la capture sélective et de l'élimination du microorganisme ou des microorganismes sans compromettre la capacité du microorgnisme ou des microorganismes de se répliquer ; le lavage du support afin d'éliminer les matières non liées ; l'addition d'un bouillon nutritif stérile au support ; l'incubation du support dans le bouillon nutritif à une température et pour une durée de temps relatives au temps de génération du microorganisme ou des microorganismes, et suffisantes afin de permettre au microorganisme ou aux microorganismes liés au support d 'atteindre un taux détectable ; le lavage du support ; suivi de la réalisation d'un essai immunologique spécifique contre le microorganisme ou les microorganismes étant détectés .

2. Procédé suivant la revendication 1 , dans laquelle les anticorps sont des anticorps élevés contre un antigène de surface du microorganisme ou des microorganismes .

3. Procédé suivant la revendication 2 , dans laquelle l'antigène de surface est une protéine flagellaire ou un lipopolysaccharide .

4. Procédé suivant l'une ou l'autre des revendications de 1 à 3 , dans lesquelles le support solide est une perle , un tube ou un réservoir .

5. Procédé suivant la revendication 4 , dans laquelle le support est du polystyrène , du chlorure de polyvynile, de l'hydroxide titaneux , des perles d'agarose ou de la nitrocellulose .

6. Procédé suivant l'une ou l'autre des revendications de 1 à 5 , dans lesquelles le microorganisme est une espèce Salmonella ou une espèce Listeria .

7. Procédé suivant l'une ou l'autre des revendications de 1 à 6 , dans lesquelles l'échantillon est exposé au support pendant une heure ou pendant moins d'une heure.

8. Procédé suivant l'une ou l'autre des revendications de 1 à 7 , dans lesquelles le support est lavé avec un tampon salin stérile .

9. Procédé suivant la revendication 8 , dans laquelle le tampon salin stérile est un tampon Tris-Salin .

10. Procédé suivant l'une ou l'autre des revendications de 1 à 9 , dans lesquelles le bouillon nutritif est un bouillon de soja tryptone ou un bouillon M .

11. Procédé suivant l'une ou l'autre des revendications de 1 à 10 , dans lesquelles l'incubation dans le bouillon nutritif a lieu pendant la nuit à une température de 37°C .

12. Procédé suivant l'une ou l'autre des revendications de 1 à 11 , dans lesquelles l'incubation dans le bouillon nutritif a lieu pendant 6 heures à une température de 37°C .

13. Procédé suivant l'une ou l'autre des revendications de 1 à 12 , dans lesquelles l'essai immunologique est un E.L.I.S.A.

14. Procédé suivant la revendication 13 , dans laquelle l'immunoréactif est un anticorps d'un antimicroorganisme avec marquage enzymatique.

15. Procédé suivant la revendication 13 , dans laquelle l'immunoréactif comprend un anticorps d'un antimicroorganisme et un anticorps avec marquage enzymatique élevés contre l'anticorps du microorganisme.

16. Procédé suivant l'une ou l'autre des revendications de 1 à 14 , un procédé qui comprend : l'exposition d'un échantillon d'essai de Salmonella pour une période d'environ 5 à environ 30 minutes à un support de polystyrène , auxquels des anticorps flagellés anti Salmonella sont adsorbés , lesdits anticorps étant capables de la capture sélective et de l'immobilisation de la Salmonella sans compromettre la capacité de la Salmonella de se répliquer ; le lavage du support afin d'enlever le matériel non lié dans une solution pour un premier lavage ; l'addition d'un bouillon nutritif au support ; l'incubation du support à 37°C pendant une période d'environ 1 heure à environ 6 heures ; le lavage du support avec une deuxième solution ; l'addition d'un anticorps avec marquage enzymatique spécifique à la Salmonella ; l'incubation pendant une période d'environ 5 à environ 30 minutes ; le rejet de tout excès d'anticorps avec marquage enzymatique ; le lavage du support avec la deuxième solution de lavage ; l'addition d'un substrat chromogénique spécifique contre l'enzyme de l'anticorps avec marquage enzymatique ; et la mesure de la conversion du substrat chromogénique en composé coloré.

17. Procédé suivant la revendication 16 , dans laquelle le support est un tube , une perle ou un réservoir microtitre .

18. Procédé suivant les revendications 16 ou 17 , dans lesquelles l'anticorps avec marquage enzymatique est un conjugué anticorps-péroxydase (anti-<u>Salmonella</u>) .

19. Procédé suivant l'une ou l'autre des revendications de 16 à 18 , dans lesquelles la solution utilisée pour le premier lavage est une solution Tris-Saline et la solution utilisée pour le deuxième lavage est une solution Tris-Saline-Tween.

20. Procédé suivant l'une ou l'autre des revendications de 16 à 19 , dans lesquelles le substrat est une solution ABTS/$H_2O_2$ .

21. Procédé suivant l'une ou l'autre des revendications de 16 à 20 , dans lesquelles le bouillon nutritif est un bouillon de soja trytone .

22. Coffret d'analyse pour la détection de bas taux d'un microorganisme particuliers ou de microorganismes dans une population mixte, ledit coffret comprenant : un support solide auquel sont adsorbés des anticorps spécifiques contre les microorganismes étant détectés , lesdits anticorps étant capables de la capture sélective et de l'immobilisation du microorganisme ou des microorganismes sans compromettre la capacité du microorganisme ou des microorganismes de se répliquer ; un anticorps avec marquage enzymatique spécifique contre le microorganisme ou les microorganismes étant détectés; ainsi qu'une solution d'un substrat chromogénique pour les enzymes des anticorps avec marquage enzymatique.

23. Coffret d'analyse suivant la revendication 22 , dans laquelle le support comprend des tubes de polystyrène enrobés d'un anticorps anti-<u>Salmonella</u> .

24. Coffret d'analyse suivant la revendication 22 ou la revendication 23 , dans laquelle l'anticorps avec marquage enzymatique comprend un conjugué anticorps-péroxydase anti-<u>Salmonella</u> .

25. Coffret d'analyse suivant les revendications 22 à 24, dans lesquelles la solution de substrat comprend du ABTS/$H_2O_2$ .

26. Coffret d'analyse pour la détection de bas taux d'un microorganisme particulier ou de microorganismes dans une population mixte, ledit coffret comprenant : un support solide auquel sont adsorbés des anticorps spécifiques contre les microorganismes étant détectés , lesdits anticorps étant capables de la capture sélective et de l'immobilisation du microorganisme ou des microorganismes sans compromettre la capacité du microorganisme ou des microorganismes de se répliquer ; un anticorps spécifique contre le microorganisme ou les microorganismes étant détectés; un anticorps avec marquage enzymatique spécifique contre le microorganisme ou les microorganismes ; ainsi qu'une solution d'un substrat chromogénique pour les enzymes des anticorps avec marquage enzymatique.